# EUROPÄISCHE PATENTANMELDUNG

(11) **EP 3 758 016 A1**
(43) Veröffentlichungstag der Anmeldung: **30.12.2020**
(21) Anmeldenummer: 19182365.7
(22) Anmeldetag: 25.06.2019
(51) Int. Cl.: G16H 40/20

(54) **VERFAHREN UND VORRICHTUNG ZUR ZUSAMMENSTELLUNG VON INSTRUMENTENSETS**

(71) Anmelder: Aesculap AG, 78532 Tuttlingen (DE)
(72) Erfinder: Mader-Ilg, Jennifer, 78532 Tuttlingen (DE); Rees, Elke, 78532 Tuttlingen (DE); Utz, Michael, 78532 Tuttlingen (DE)

(57) **Zusammenfassung**

Die vorliegende Erfindung betrifft ein computer-implementiertes Verfahren zur Zusammenstellung von Instrumentensets für eine Operation mit den Schritten Eingeben von Konfigurationsparametern, Berechnen von mindestens einer Konfiguration unter Berücksichtigung der Konfigurationsparameter, Anzeigen der berechneten Konfigurationen, Rekonfigurieren der berechneten Konfiguration im Ansprechen auf empfangene Eingaben durch Modifikationen die den Eingaben entsprechen, und Prüfen der rekonfigurierten Konfiguration hinsichtlich vorab definierter Bedingungen.

## Beschreibung

### GEBIET DER ERFINDUNG

Die vorliegende Erfindung betrifft im Allgemeinen die Zusammenstellung von Instrumentensets, und insbesondere die Zusammenstellung von medizinischen Instrumenten in Sätzen, so dass diese optimiert sind für verschiedene Einsatzzwecke oder nach verschiedenen Parametern.

### HINTERGRUND DER ERFINDUNG

Die folgende Beschreibung des Standes der Technik dient dazu die Erfindung und insbesondere die Vorteile der Erfindung besser verstehen zu können. Es versteht sich, dass jegliche Darlegung des Standes der Technik weder explizites noch implizites Zugeständnis ist, dass dieser Stand der Technik weithin bekannt ist oder Allgemeinwissen auf dem Gebiet der Erfindung darstellt.

Gegenwärtig werden bei Operationen, z.B. Hüft-, Knie- oder Wirbelsäulenoperationen, in Kliniken und Krankenhäusern standardisierte Instrumentensets verwendet. Diese beinhalten in der Regel einen oder mehrere Sätze von Instrumenten, die bei der jeweiligen Operation zum Einsatz kommen könnten. Einen Satz von Instrumenten nennt man auch Instrumentenset.

Gelegentlich gibt es für dieselbe Operation verschiedene Varianten. Dabei können sich zu bereitstellende Instrumentensets unterscheiden. Diese Unterschiede können z.B. im Typ des Implantats begründet sein, seiner Bauform, z.B. Implantattyp mit Langschaft oder Kurzschaft, oder auch in einer Bestimmten Version, d.h. Revision, des Implantats. Einzusetzende Instrumente können auch vom Hersteller des Implantats abhängen. In anderen Worten, die Werkzeuge, d.h. Instrumente, die bei der Operation, die ein Implantat beinhaltet, zur Anwendung kommen könnten, könnten sich je nach Implantat unterscheiden. Dies kann auch begründet sein in der verschiedenen Ausgestaltung der Implantate, z.B. welche Raspeln oder Handgriffe eingesetzt werden. Auch gibt es in Anpassung an die Patienten verschiedene Größen der jeweiligen Implantate, was ebenfalls auf die Wahl der Instrumente Einfluss nimmt. Die Instrumentensets beinhalten also implantatsspezifische Instrumente, die für eine Vielzahl an Varianten der jeweiligen Operation benötigt werden könnten.

Doch auch bei anderen Operationen, d.h. Operationen, die keine Implantate beinhalten, können unterschiedliche Instrumentensets zum Einsatz kommen, z.B. je nach Vorlieben des operierenden Arztes. Des Weiteren können sich Instrumente nach geographischer Region des Einsatzes unterscheiden, z.B. bedingt durch gesetzliche oder andere Vorschriften.

Generell gilt, dass in der Regel mehr Instrumente in den jeweiligen Instrumentensets bereitgestellt werden, damit die Fachkräfte, die an der Operation beteiligt sind, für alle Eventualitäten einer Operation vorbereitet sind. Es können nämlich jederzeit während einer Operation ungeplante Ereignisse vorkommen, die dann zusätzliche Instrumente erfordern. Diese Ereignisse können medizinisch bedingt sein, oder auch lediglich dadurch bedingt, dass ein Instrument zu Boden fällt und somit nicht mehr steril ist. Es liegen in der Regel umfangreiche Erfahrungswerte vor, so dass die meisten Eventualitäten berücksichtigt werden können.

Die Instrumente, die für eine Operation vorgesehen sind, sind in fest montierten Halterungen gelagert, die wiederum in Siebkörben verankert sind. So gelagert, werden die Instrumente häufig gewaschen und sterilisiert bzw. nach dem Waschvorgang eingeräumt und anschließend darin immer wieder erneut sterilisiert.

Diese Halterungen, auch Lagerungen oder Lagerungselemente genannt, sind fest mit einem Korb verbunden und können nicht verändert werden, ohne durch massiven Werkzeugeinsatz und dass dadurch ein neues Instrumentenset entsteht. Der Inhalt der Siebkörbe und deren Anordnungen darin sind somit fest vorgegeben.

Durch die zuvor geschilderten Unterschiede hinsichtlich bevorzugten operativen Vorgehens sowie die Auswahl von Implantaten hinsichtlich Größe und Typ können sich die benötigten Instrumente und deren bevorzugte Anordnung jedoch deutlich unterscheiden. Bisherige Instrumentensets enthalten aufgrund der oben dargestellten Situation also Instrumente, welche selten oder nie benötigt werden, und die somit die Siebkörbe unnötig schwer machen. Sie nehmen auch zusätzlichen Platz ein, so dass möglicher Weise zusätzliche Siebkörbe verwendet werden müssen. Das Materialvolumen wiederum hat direkten Einfluss auf die benötigten Kapazitäten beim gesamten Wiederaufbereitungsprozess. Der chemische Aufbereitungsprozess beeinflusst die Qualität der Instrumente negativ, was besonders unnötig erscheint, wenn diese nicht zum Einsatz gekommen sind. Es ist außerdem bekannt, dass eine höhere Anzahl von Instrumenten die Tätigkeit der instrumentierenden Personen, z.B. OP-Schwester, verkompliziert und dadurch ein höherer zeitlicher Aufwand anfällt, um die richtigen Instrumente in einem Sieb zu suchen.

Ein weiterer Aspekt sind die Kosten bei der Ausstattung. Chirurgische Instrumente bestehen häufig aus komplexen mechanischen Baugruppen, welche entsprechend hohe Herstellkosten mit sich bringen. Werden solche teuren Instrumente häufig zur Verfügung gestellt, obwohl sie nur in einzelnen Fällen benötigt werden, entsteht unnötiger Aufwand bei Transport, Verpackung, Versand, und Reinigung. Eine Optimierung dieser Parameter birgt enormes Einsparungspotential für den Hersteller und Anwender.

Bekannt sind modulare Lagerungslösungen, bei denen die Instrumente in verschiedenen Gruppen zusammengestellt werden, aus denen jeweils die benötigten Gruppen bei einer Bestellung zusammen bestellt werden können. Diese bekannten Lagerungslösungen sind aber nicht geeignet zur Individualisierung. Es gibt bisher kein Verfahren, was die optimierte Zusammenstellung von Instrumentarien nach Anwendervorgaben unterstützt. Die bekannten Lösungen verursachen hohen Aufwand für die Bereitstellung ungenutzter Instrumente und Implantate beim Hersteller, wie oben ausgeführt. Auch in den Kliniken entsteht erhöhter Aufwand durch die notwendige, wiederholte Aufbereitung ungenutzter Instrumente und Lagerungen und auch durch die erhöhte Komplexität beim Bereitstellen der Instrumente auf Instrumententischen. Die häufige Aufbereitung nicht genutzter Instrumente geht mit einem entsprechenden Qualitätsverlust einher. Die bekannten Lagerungen sind nicht an die Bedürfnisse und Prozesse des Anwenders angepasst, wodurch sie unübersichtlicher als bei einer individualisierten Ausstattung sind.

Die Aufgabe der Erfindung ist es die oben beschriebenen Nachteile des Standes der Technik zu überwinden, den Stand der Technik zu verbessern, und/oder wenigstens eine nützliche Alternative zum bestehenden Stand der Technik anzubieten.

### ZUSAMMENFASSUNG DER ERFINDUNG

Die vorliegende Erfindung löst die obengenannte Aufgabe durch die Gegenstände der unabhängigen Patentansprüche. Vorteilhafte Weiterbildungen finden sich in den jeweiligen abhängigen Patentansprüchen.

Insbesondere löst die vorliegende Erfindung die Aufgabe durch Erfassen von Informationen über anwenderspezifische Prozesse, Abläufe und Vorlieben. Diese Informationen werden mit rechtlichen und/oder regulatorischen Vorgaben abgeglichen und nötigenfalls angepasst. Weiterhin werden die Informationen mit Informationen über das Instrumentarium verglichen, die bereits vorab gespeichert wurden. Danach kann ein erneuter Abgleich mit rechtlichen und/oder regulatorischen Vorgaben erfolgen. Schließlich werden die Informationen verwertet durch die Erstellung der optimalen Konfiguration hinsichtlich des verfügbaren Instrumentariums, der anwenderspezifischen Prozesse, Abläufe und Vorlieben, sowie der rechtlichen und/oder regulatorischen Vorgaben.

Die erstellte optimale Konfiguration kann ausgegeben werden, und daraufhin weitere Modifikationen im Rahmen einer Rekonfiguration erlauben.

Danach kann ein erneuter Abgleich mit rechtlichen und/oder regulatorischen Vorgaben erfolgen, sowie ein erneuter Vergleich mit den Informationen über das Instrumentarium durchgeführt werden.

Optional kann die erstellte Konfiguration auch unter Berücksichtigung des Reinigungsvorganges optimiert werden.

Optional kann zusätzlich zur erstellten Konfiguration eine Anleitung erstellt werden, die die Reihenfolge der zu verwendenden Instrumente angibt. Die Anleitung kann in einer beliebigen Sprache erstellt werden und kann optional Bilder enthalten.

Die erstellte, optimale Konfiguration kann sodann in eine Zusammenstellungsvorrichtung weitergeleitet werden, welche automatisiert die erstellte Konfiguration umsetzt und die Siebkörbe in der erstellten Konfiguration zusammenstellt.

Die Vorteile der Erfindung bestehen in geringeren Aufbereitungskosten beim Anwender, z.B. in den Kliniken, durch potenziell reduzierte Anzahl an Instrumenten und Siebkörben. Des Weiteren werden die Operationsabläufe durch Reduktion und Anordnung der Instrumente in Abstimmung mit den Prozessen und Prozessschritten vereinfacht und sicherer gemacht. Ein zeitlicher Vorteil vor und während der Operation führt zur Reduktion der Operationsrisiken, da durch die optimierte Lagerung gemäß dem Ablauf der Operation die Suche nach Instrumenten entfällt und die Stressbelastung der Beteiligten sinkt.

### KURZBESCHREIBUNG DER ZEICHNUNG

Die obenstehende Zusammenfassung, sowie weitere Aufgaben, Merkmale und Vorteile der vorliegenden Erfindung werden im Weiteren durch und mit Bezug auf die folgenden Figuren der Zeichnung erläutert, um so ein besseres Verständnis der Erfindung zu ermöglichen. Darin bezeichnen gleiche Bezugszeichen gleiche Elemente.

Es zeigen:
die Figuren 1 bis 3 beispielhafte Siebkörbe mit Instrumenten;
die Figuren 4 bis 19 beispielhafte Eingabemasken einer Implementierung der Erfindung; und
die Figur 20 ein Flussdiagramm des erfinderischen Verfahrens.

### AUSFÜHRLICHE BESCHREIBUNG

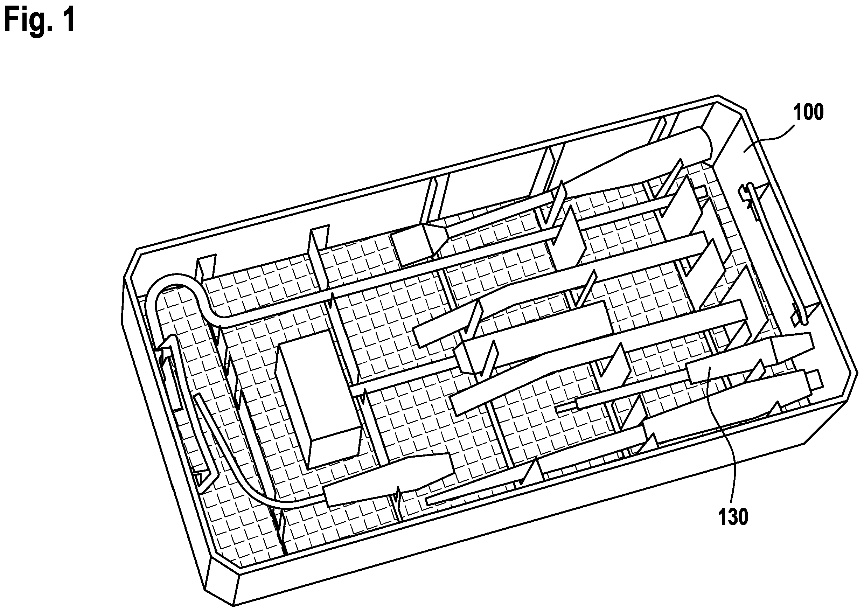

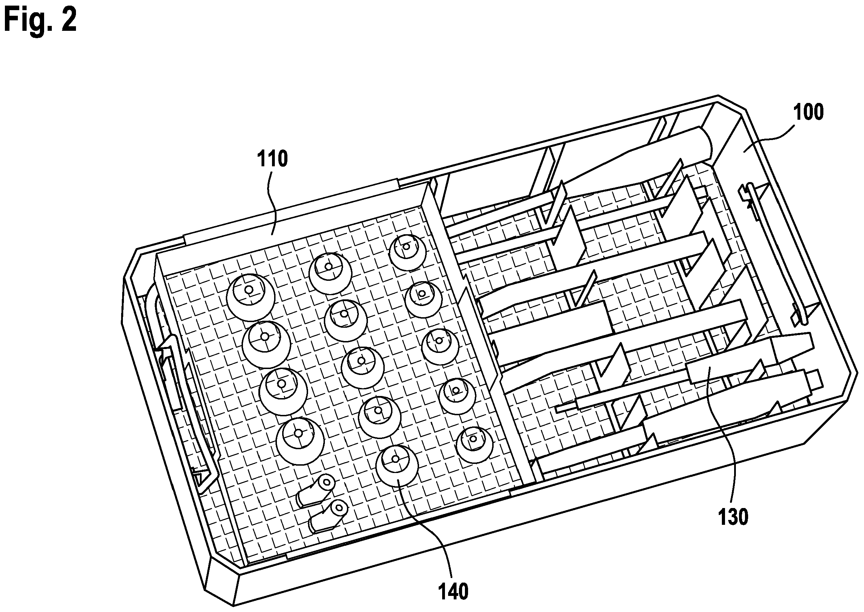

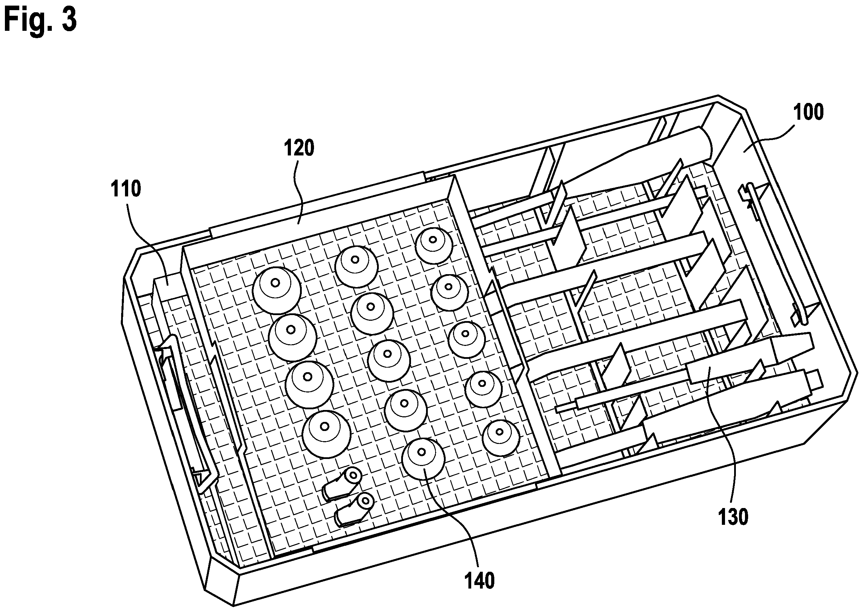

Figur 1 zeigt einen beispielhaften Siebkorb 100. Figuren 2 und 3 zeigen den beispielhaften Siebkorb 100 mit Einhängern 110 und 120. In dem Siebkorb und den Einhängern sind Instrumente 130 und Zubehör 140 abgebildet. Zubehör 140 kann auch im Siebkorb angeordnet werden, ebenso wie Instrumente 130 in Einhängern angeordnet werden können.

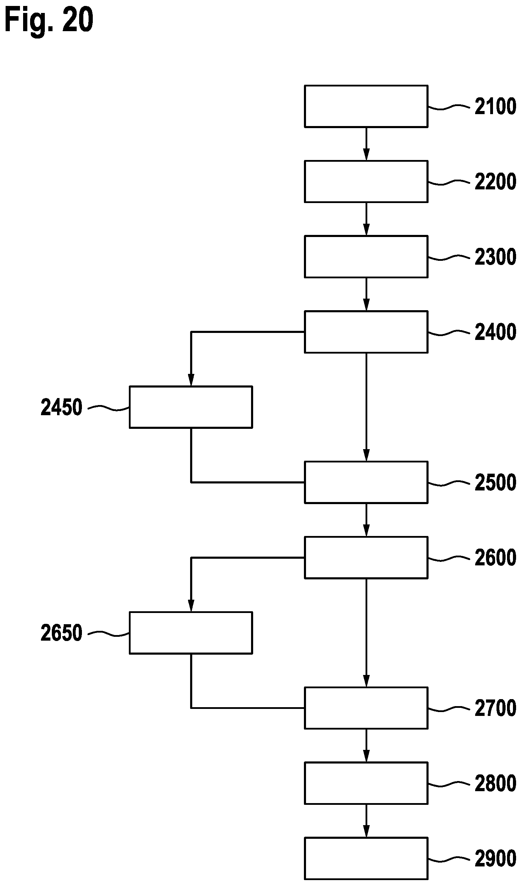

Unter Bezugnahme auf Figur 20 wird im Folgenden das erfinderische Verfahren zur Zusammenstellung von Siebkörben beschrieben. Es wird im Weiteren lediglich von einem Siebkorb die Rede sein, es versteht sich jedoch, dass je nach Menge der benötigten und gewünschten Instrumente, die fertige Konfiguration mehr als einen Siebkorb umfassen kann.

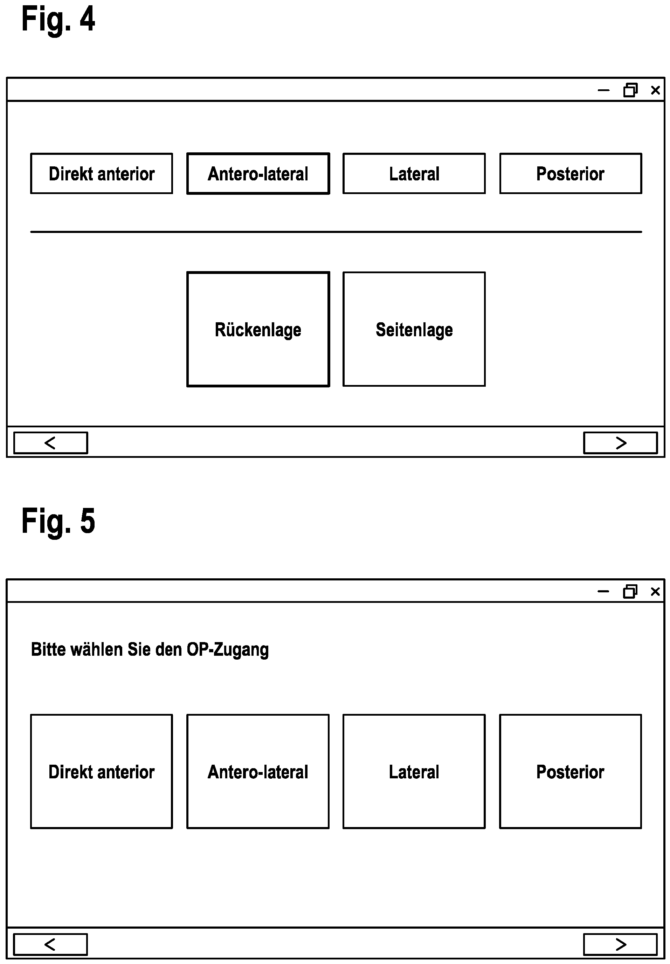

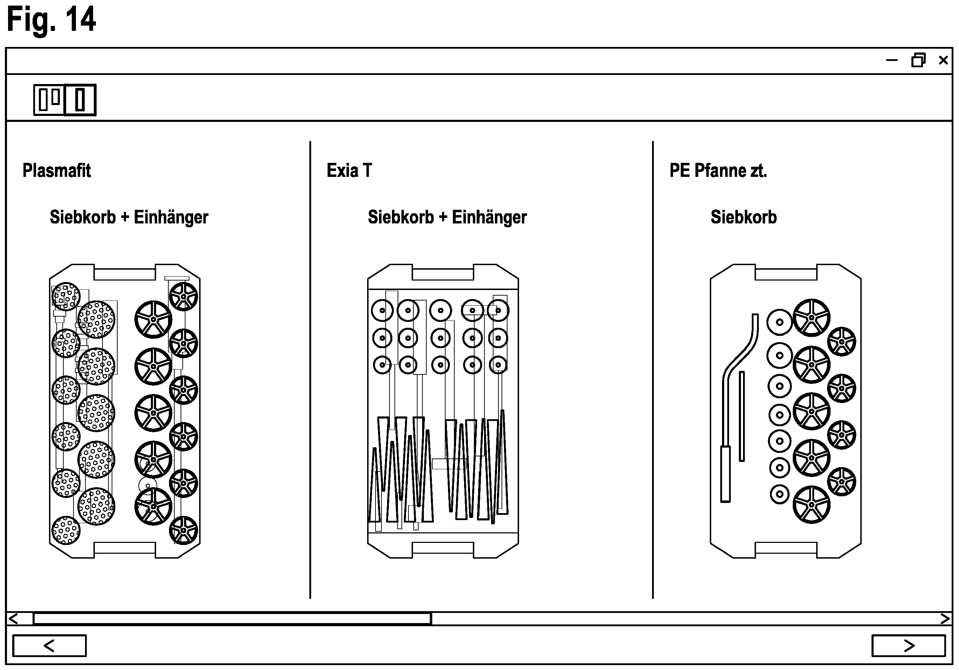

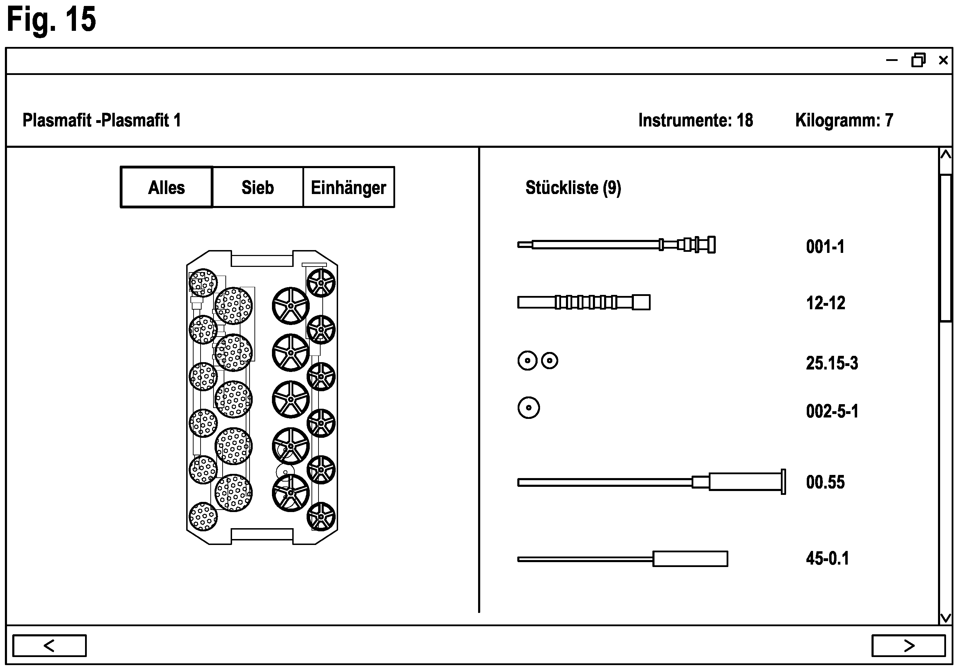

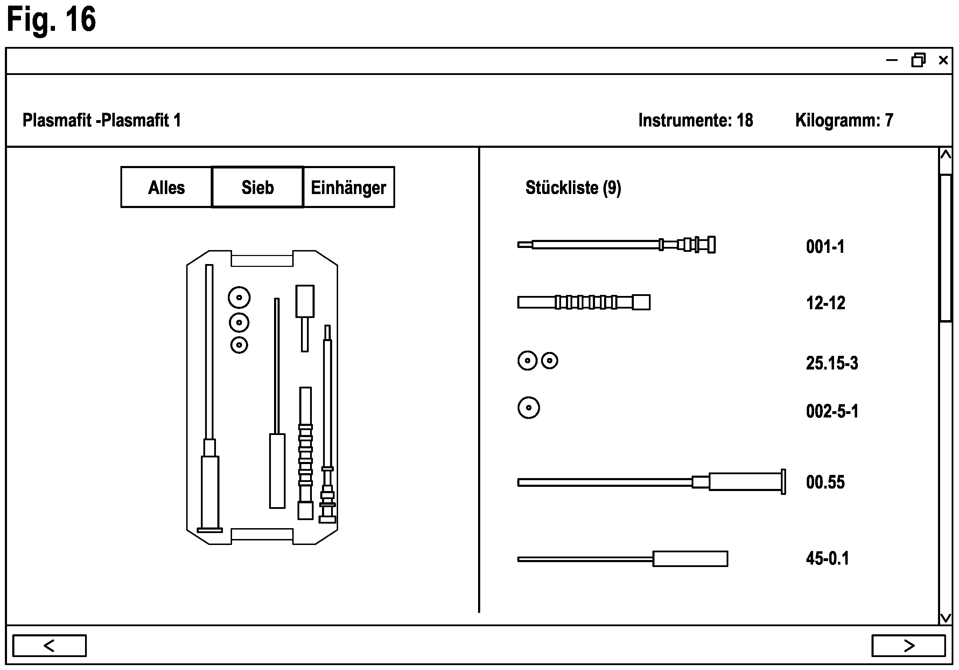

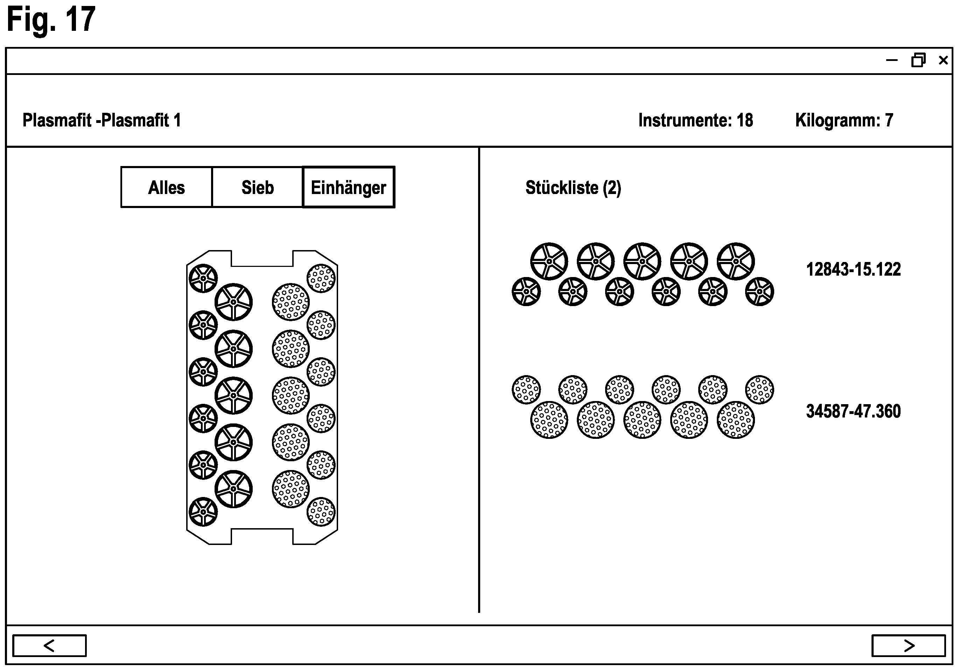

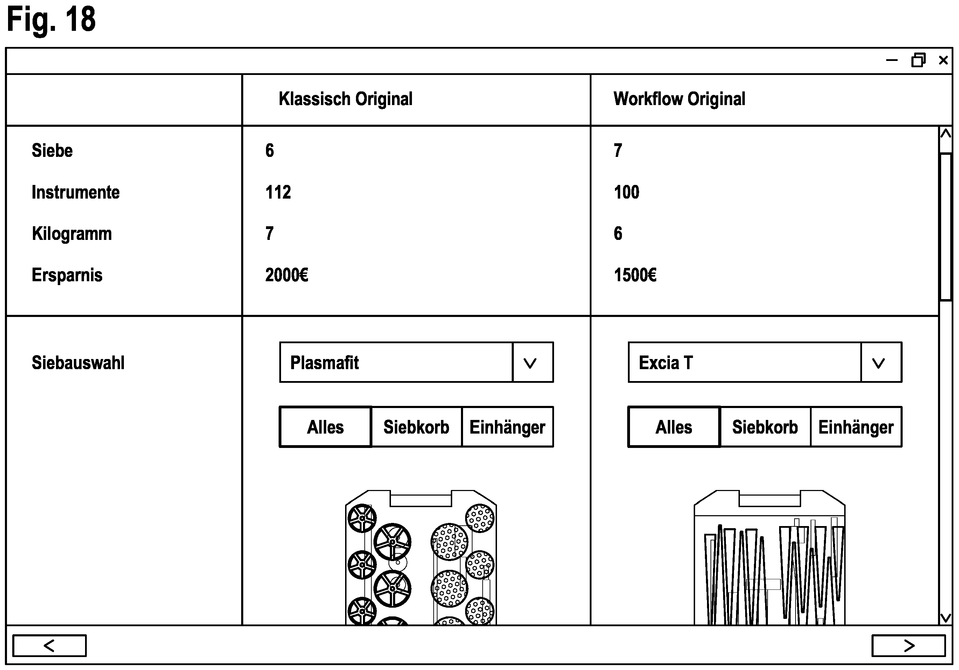

Dabei ist anzumerken, dass die Eingabe der Informationen lediglich beispielhaft anhand der Eingabemasken der Figuren 4 bis 19 erläutert wird. Eine andere Art der Datenerfassung ist ebenso denkbar, z.B. ohne grafische Oberflächen. Ebenso ist selbstverständlich, dass vorkonfigurierte Informationen bereitgestellt werden können, sowie fertige Konfigurationen gespeichert und geladen werden können. Dieses Speichern fertiger Konfigurationen kann im Falle einer Client-/Serverarchitektur, wie z.B. beim Bereitstellen der Eingabemöglichkeiten über das Internet oder andere Netzwerke, sowohl auf dem Client als auch auf dem Server erfolgen.

In Schritt 2100 kann also eine bestehende Konfiguration geladen werden oder eine neue Konfiguration angelegt werden. Für den Fall, dass eine bestehende Konfiguration geladen wird, sind die folgenden Schritte als Überprüfungs- und/oder Modifikationsschritte zu verstehen. Zu Beginn der Konfiguration oder nach Laden einer bestehenden Konfiguration kann auch die Orientierung der Instrumente ausgewählt werden. Diese Orientierung kann der Vorliebe des Operateurs entsprechen oder auch durch die Entnahmeseite, die sich von Rechtshändern zu Linkshändern unterscheidet, bestimmt sein.

In Schritt 2200 kann eine Patientenlagerung eingegeben werden. Eine entsprechende Eingabemaske ist in Fig. 4 dargestellt. Je nach durchzuführender Operation muss der Patient auf dem Rücken, auf einer Seite oder auf der Brust liegend gelagert werden. Bei einigen Operationen ist mehr als eine Lagerung möglich und der Anwender kann entscheiden welche Lagerung berücksichtigt werden soll. Es ist außerdem möglich mehr als eine Lagerung auszuwählen, so dass im fertigen Siebkorb alle Instrumentarien vorgehalten werden, um dem Operateur die letzte Entscheidung bezüglich der Patientenlagerung zu überlassen.

In Schritt 2200 kann außerdem der Patientenzugang erfasst werden. Eine entsprechende Eingabemaske ist in Fig. 5 dargestellt. Je nach Operation muss ein bestimmter Zugang gewählt werden oder es besteht eine Auswahl der Zugangswege. Analog zur Patientenlagerung kann hier ein oder mehrere Zugänge ausgewählt werden, so dass das entsprechende Instrumentarium in der fertigen Konfiguration berücksichtigt wird. Darüber hinaus können weitere patienten- oder operateurspezifische Parameter definiert werden, um so eine auf die Anwenderbedürfnisse abgestimmte Instrumentenkonfiguration zu erstellen.

Die Erfassung des Zugangs und der Patientenlagerung können in beliebiger Reihenfolge oder auch zeitgleich im Schritt 2200 ausgeführt werden.

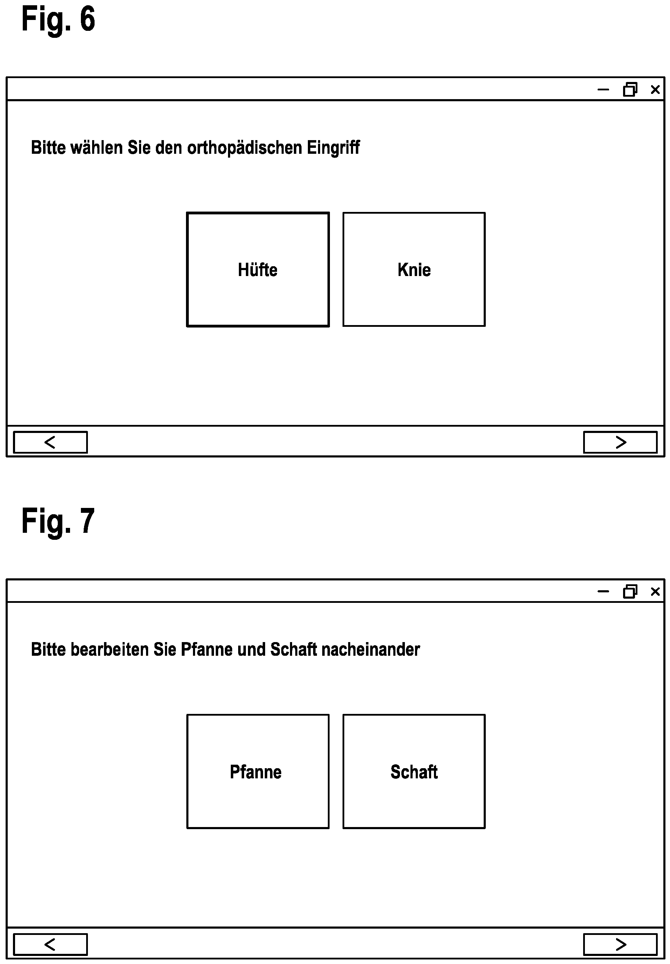

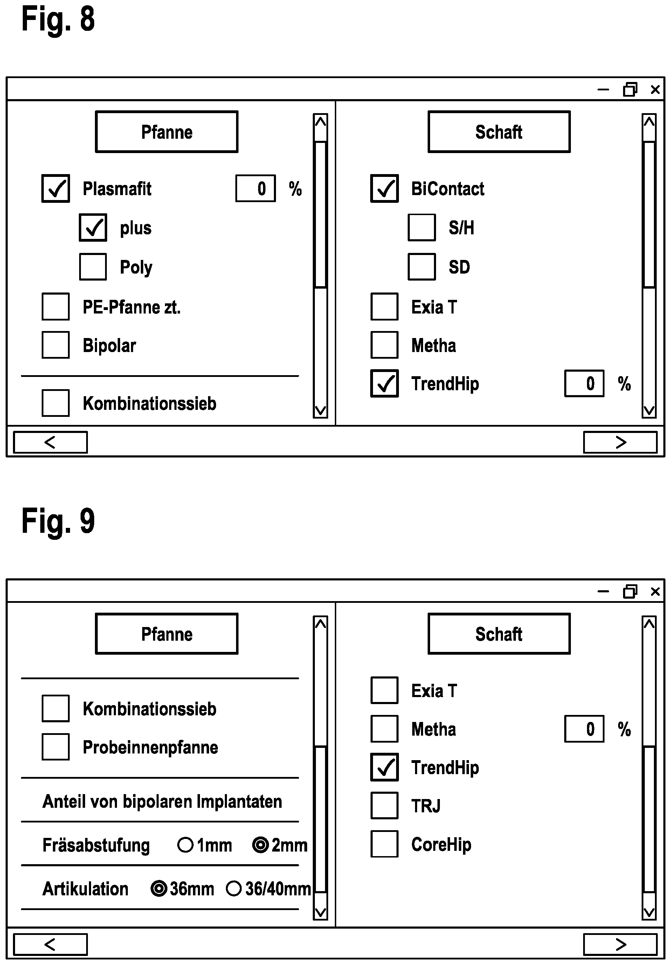

Im Schritt 2300 wird der Eingriff und das zu verwendende Implantat gewählt. Entsprechende Eingabemasken sind in Figuren 6 und 7 dargestellt. Durch die Wahl des Implantats erfolgt automatisch die Zuwahl von durch das Implantat bedingten Instrumentarien sowie entsprechendes notwendiges Zubehör. Gibt es für Implantate, Instrumente und Zubehör alternative Möglichkeiten, so werden diese Alternativen zur Auswahl gestellt. Entsprechende Eingabemasken sind in Figuren 8 und 9 dargestellt. Alternative Möglichkeiten können durch verschiedene Hersteller, verschiedene Werkstoffe, verschiedene Größen, oder andere Parameter bestimmt sein.

Schritt 2300 kann auch vor Schritt 2200 ausgeführt werden.

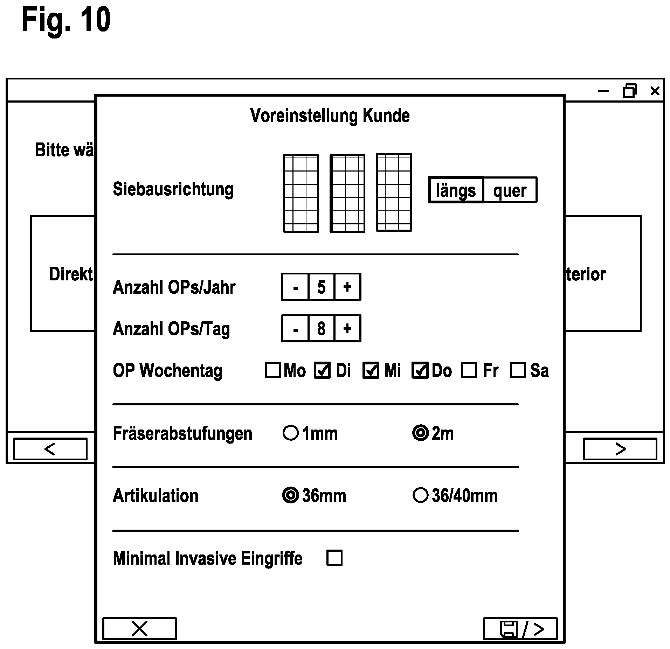

In Schritt 2400 werden sodann Vorschläge für die Konfiguration auf der Grundlage der eingegebenen Parameter sowie der dadurch verknüpften und bereits gespeicherten Parameter berechnet. Eine entsprechende Eingabemaske ist in Fig. 10 dargestellt. Bei der Berechnung der Vorschläge können beispielsweise folgende Bedingungen berücksichtigt werden. Alle diese Eigenschaften sind in einer Datenbank für jedes Element der Gesamtheit der Instrumentarien hinterlegt.

Durch die Auswahl der jeweiligen Patientenlagerungen und Zugangswege werden bestimmte aus der Gesamtheit der Instrumentarien verfügbare Elemente gefiltert, da diese für die ausgewählten Patientenlagerungen bzw. Zugänge nicht zu verwenden sind.

Des Weiteren werden die zuvor definierten Kundenvoreinstellungen berücksichtigt, wie etwa die Orientierung der Instrumente im Siebkorb. Die Siebausrichtung beeinflusst die Orientierung der Instrumente im Siebkorb. Die Anordnung soll so sein, dass die Entnahme der Instrumente bequem erfolgen kann und bei Instrumenten die in der Orientierung sowohl hochkant als auch quer im Siebkorb liegen können, die Ausrichtung sich entsprechend mit der Siebausrichtung ändert.

Ebenso sind die jeweiligen Platzbedarfsparameter für die Instrumente und weiteren Elemente gespeichert, so dass der Bauraum und Spülraum pro Instrument bei der Anordnung ermittelt werden kann, woraus sich der Gesamtplatzbedarf und somit die Anzahl der Siebkörbe ergibt. Hierbei ist der Bauraum derjenige Raum, den ein Instrument inklusive konstruktiv bedingter Abstände in der endgültigen Lagerung einnimmt. Der Spülraum ist derjenige Raum, den das Instrument inklusive freizulassender Räume einnehmen muss, damit die maschinelle Reinigung der Instrumente in den Siebkörben gewährleistet werden kann. Somit kann die Lagerung auch als Waschlagerung genutzt werden, was die Wiederaufbereitung deutlich vereinfacht. Der Spülraum der einzelnen Instrumente kann sich ggf. überlagern, jedoch nicht der Bauraum. Hierdurch kann eine Optimierung der Siebbestückung durch die bestmögliche Ausnutzung des Raumangebots zur Lagerung der Instrumente erreicht werden. Ein Packen nach dem Reinigungsprozess ist nicht notwendig, dadurch kommt es zu einer erheblichen zeitlichen Ersparnis in der Zentralen Sterilgutversorgungsabteilung (ZSVA) des Anwenders.

Die Patientenlagerung, der operative Zugang und die Auswahl von Implantaten bestimmen auch welche Instrumente dem Anwender zur Konfiguration vorgeschlagen werden und welche komplett ausgeblendet werden.

Die anwenderspezifischen Informationen werden zur Ermittlung der notwendigen Anzahl von Instrumenten und Implantaten sowie zur Kostenkalkulation herangezogen. Darüber hinaus werden sie direkt in vorhandene CRM und Warenwirtschaftssysteme übertragen. Damit ist eine Transparenz auf Anwenderebene gewährleistet. Modulare Lagerungselemente ermöglichen die Umsetzung der geplanten anwenderspezifischen Setkonfiguration. Modulare Lagerungselemente ermöglichen weiterhin die Realisierung der Verschiedenen Konfigurationen unter jeweils einer Artikelnummer.

Ebenso hat das Gewicht jedes Elementes der Instrumente einen Einfluss auf die Anordnung und somit auch auf die Anzahl der Siebkörbe.

Für bestimmte Implantate können ein oder mehr Instrumente zwingend vorgeschrieben sein, so dass diese auf jeden Fall in der fertigen Konfiguration enthalten sein müssen. Für andere Implantate kann vorgeschrieben sein, dass eines einer Gruppe von Alternativen verwendet wird, so dass je nach Auswahl durch den Anwender, auf jeden Fall ein Instrument der Gruppe in der fertigen Konfiguration enthalten sein muss. Zusätzlich können chirurgische Standardinstrumente, ebenso auf Basis der zuvor genannten Informationen vorausgewählt und dem Instrumentarium hinzugefügt werden.

Darüber hinaus kann auch eine zeitliche Anforderung an die Instrumente gespeichert sein, die z.B. angeben kann in welcher Reihenfolge und in welchem der Operationsschritte ein Instrument benötigt wird. Für jedes Instrument kann außerdem hinterlegt sein, welche Instrumente miteinander kombiniert werden können, so dass für solche mehrteiligen Instrumententeile entweder eine automatische Zuwahl bei nur einer Option oder eine Auswahl bei mehreren Alternativen erfolgen kann.

Da die Bestückung der Siebkörbe dreidimensional erfolgen kann, ist auch die Bauhöhe hinterlegt und welche Instrumente in einer zweiten Ebene gelagert werden können und welche Instrumente die Möglichkeit bieten, darüber eine zweite Ebene zu lagern. Eine zweite oder weitere Ebene kann durch sogenannte Einhänger realisiert werden, wie in den Figuren 2 und 3 dargestellt. Hierbei ist in Figur 2 eine Konfiguration mit einem Einhänger 110 dargestellt und in Figur 3 eine Konfiguration mit zwei Einhängern 110 und 120 dargestellt. Weitere Ebenen, die durch weitere Einhänger realisiert werden sind vorstellbar.

Des Weiteren wird berücksichtigt, dass es für alle in einem Siebkorb angeordneten Elemente mindestens ein gemeinsames Reinigungs-, Desinfektions-, und Sterilisationsverfahren (RDS-Verfahren) gibt.

Schließlich wird im Sinne der Optimierung der Anordnung der Elemente im jeweiligen Siebkorb versucht, die Siebe möglichst kompakt zu bestücken, um möglichst viel zusammenhängenden Freiraum zu bekommen, was sich vorteilhaft auf die Reinigung auswirkt.

Optional können in Schritt 2400 nicht nur eine optimale Konfiguration ermittelt werden, sondern vielmehr eine Auswahl von Konfigurationen ermittelt werden, die in einer jeweiligen Hinsicht als optimal berechnet werden. Einige Beispiele solcher Optimierungsparameter sind Größe, Reihenfolge und Umfang, jedoch sind auch andere Optimierungsparameter vorstellbar.

Bei einer Optimierung nach Größe werden in einem Hauptsiebkorb die Instrumente angeordnet, welche nötig sind um den Großteil einer Operation, z.B. 80%, abzudecken. Instrumente, die für die restlichen z.B. 20% der Operationen notwendig sind, werden in separate Siebe ausgelagert.

Bei einer Optimierung nach Reihenfolge, werden die Instrumente streng nach der Reihenfolge ihrer Verwendung während der Operation sortiert. Die Operationsabläufe können die Anordnung der Instrumente beeinflussen, da sie in der benötigten Reihenfolge berücksichtigt werden. Ein Beispiel dafür ist eine Operation am Knie, bei der z.B. die Abfolge der Knochenschnitte festgelegt sein kann. Beispiele für solche Festlegungen sind z.B. Femur-First, Tibia-First oder Gap-Balancing Technique. Durch eine am Operationsablauf orientierte Lagerung der Instrumente in den Sieben wird die Auffindung des nächsten erforderlichen Instruments erleichtert, wodurch vor und während der Operation Zeit gespart wird, was zu einer Verminderung der Risiken bei Operationen führt.

Bei einer Optimierung nach Umfang, wird versucht im Vergleich zu bestehenden Standardkonfigurationen möglichst wenig abzuweichen, jedoch wird versucht den Umfang der Instrumente aber auf das Notwendige zu reduzieren.

Unabhängig von der Art der Optimierung können auch die Instrumentensets so aufgeteilt werden, dass die Instrumente, die für jeden Implantattyp gleich sind, auch generelle Instrumente genannt, separat gelagert werden von denen, die für jeden Implantattyp unterschiedlich sind, diese werde auch spezifische Instrumente genannt. Von den spezifischen Instrumenten finden sich dann nur diejenigen im Siebkorb wieder, die zu dem gewählten Instrument gehören.

Dabei können die generellen und spezifischen Instrumente gemeinsam in einem Siebkorb-Set entsprechend, d.h. voneinander getrennt, angeordnet werden, oder es werden Teilsets bestückt, ein Teilset mit den generellen Instrumenten und ein Teilset mit den spezifischen Instrumenten, so dass dann das Teilset, das die zum gewühlten Implantat passenden spezifischen Instrumente hat zum Teilset mit den generellen Instrumenten hinzugefügt werden kann. Dies kann dann durch einen teilbaren Siebkorb oder durch Einhänger realisiert werden.

Somit kann pro Operation ein Set genereller Instrumente mit den für die Operation vorgesehenen spezifischen Instrumente kombiniert werden. Und dadurch lässt sich die Zahl der benötigten teuren Instrumente, die für alle Implantattypen identisch sind, deutlich reduzieren.

Abschließend kann optional eine Kenngröße ermittelt werden, die die Einsparung hinsichtlich der Standardsiebkörbe darstellt. Diese Darstellung kann als Platzersparnis, Gewichtsersparnis, Kostenersparnis oder Ähnliches erfolgen.

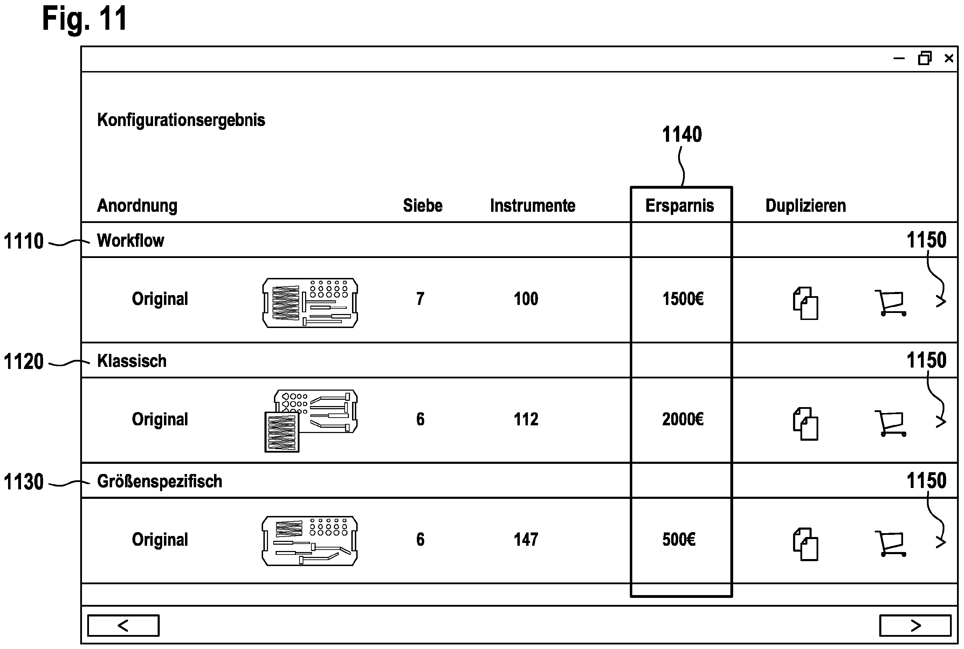

In Schritt 2500 können die durch die Berechnung erzeugten Konfigurationen angezeigt werden. Eine entsprechende Ausgabe ist in Fig. 11 dargestellt. Sollten mehrere Konfigurationen 1110, 1120, 1130 erzeugt worden sein, können diese vergleichend dargestellt werden. Für jede erzeugte Konfiguration kann darüber hinaus die errechnete Ersparnis 1140 hinsichtlich eines oder mehrerer Parameter - wie oben beschrieben - dargestellt werden.

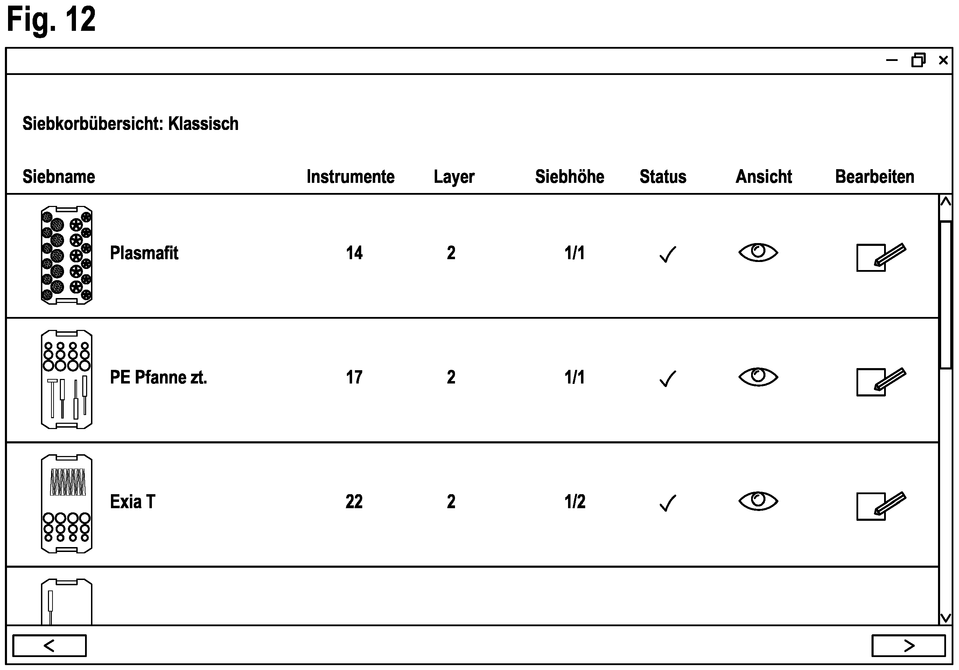

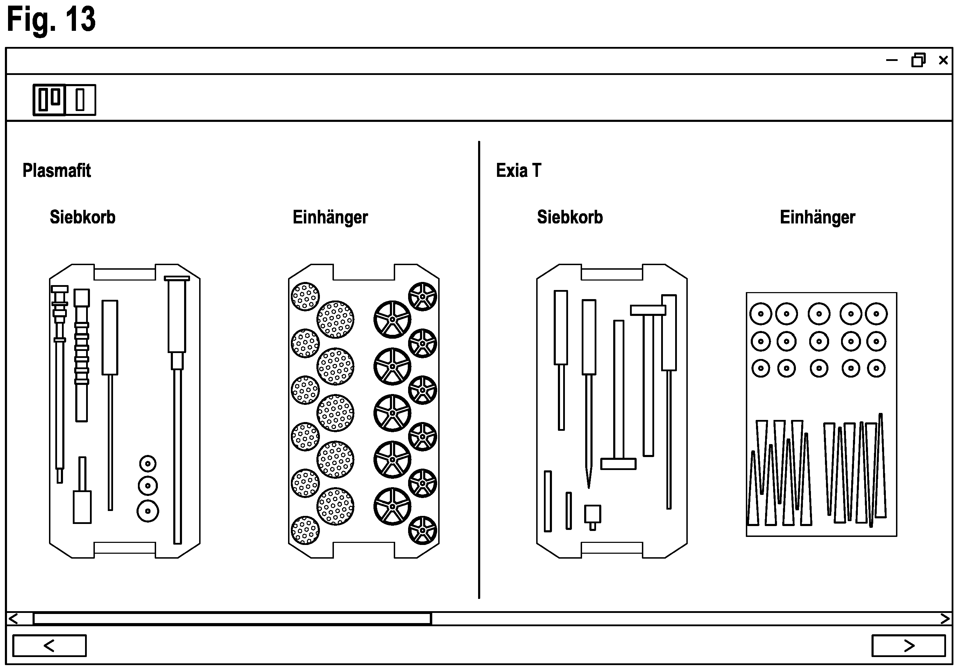

In dieser Ansicht kann darüber hinaus z.B. mittels Schaltflächen 1150 jede Konfiguration untersucht werden, was zu einer Darstellung wie in Fig. 12 dargestellt führen kann, so dass einzelne Siebkörbe oder einzelne Siebe dargestellt werden. Dies ist beispielhaft in den Figuren 13 bis 18 dargestellt.

Alle Darstellungen können grafisch oder als Stücklisten realisiert werden.

Abschließend kann im Falle mehrerer Konfigurationen eine vom Anwender ausgewählt werden oder es kann mit einer Konfiguration die automatisch nach einem vorkonfigurierten Kriterium ausgewählt wird fortgefahren werden. Solch ein vorkonfiguriertes Kriterium kann z.B. beste Ersparnis, geringste Modifikation zu Standard Siebkörben oder geringste Anzahl von Siebkörben sein. Andere Kriterien sind ebenfalls vorstellbar.

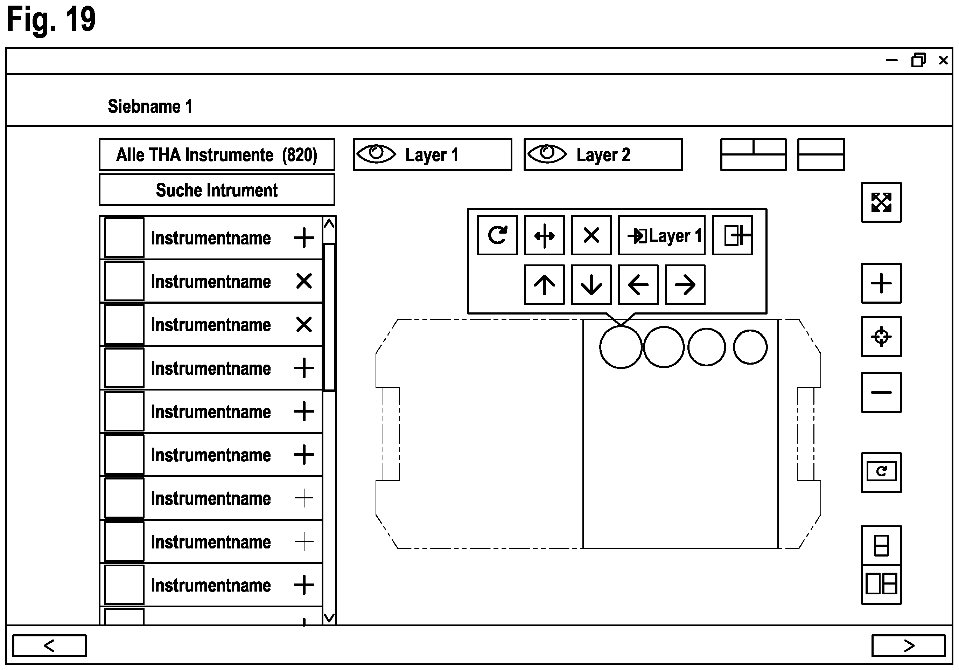

Im Anschluss daran kann in Schritt 2600 die Rekonfiguration ermöglicht werden. Eine entsprechende Eingabemaske ist in Fig. 19 dargestellt. Hierbei können die Instrumentenanordnungen und Siebkonfigurationen dem Anwender angezeigt und, auf dessen Eingaben hin, für ihn individuell modifiziert werden. Dabei werden Bauraum und Spülraum beachtet. Die Anpassungen können sowohl am Inhalt jeder Lagerung, d.h. Sieb, Siebkörbe und Einhänger, als auch an der Position und Orientierung einzelner Instrumente oder Instrumentenblöcke in der Lagerung vorgenommen werden. Bei der Rekonfiguration der Siebkörbe können unter anderem die folgenden Punkte geändert werden, bzw. müssen die folgenden Bedingungen eingehalten werden. Die für die Rekonfiguration notwendigen Eingaben können vom System als Eingaben empfangen werden.

Es kann die Orientierung der Instrumente geändert werden, sofern im jeweiligen Siebkorb möglich aufgrund der Abmessungen inkl. Bauraum für die Halterungen und Spülraum für die Reinigung. Der Bauraum und Spülraum pro Instrument darf durch die Änderungen unter Berücksichtigung des Platzbedarfs nicht unterschritten werden. Die Änderungen dürfen das Gewicht der Instrumente pro Siebkorb nicht überschreiten. Instrumente die zwingend vorgesehen werden müssen, dürfen nicht entfernt werden. Instrumente, die aus einer Gruppe stammen von denen mindestens eines enthalten sein muss dürfen gegen andere Instrumente aus der Gruppe ausgetauscht werden, das Instrument darf aber nicht entfernt werden. Die Kombinationsfähigkeit von Instrumenten wird berücksichtigt.

Ist die Konfiguration nach Reihenfolge optimiert, so darf die Modifikation diese Reihenfolge nicht ändern. Die Instrumente dürfen darüber hinaus nicht so modifiziert werden, dass ein Instrument, das nicht in der zweiten Ebene gelagert werden kann dort einkonfiguriert wird, oder dass über einem Element, über dem keine zweite Ebene angelagert werden kann, ein weiteres Element vorgesehen wird. Abschließend muss auch für die modifizierte Konfiguration gelten, dass es für alle in einem Siebkorb angeordneten Elemente mindestens ein gemeinsames Reinigungs-, Desinfektions-, und Sterilisationsverfahren (RDS-Verfahren) gibt.

Wird eine Konfiguration modifiziert, so dass es zu einer nicht erfüllten Abhängigkeit wie oben beschrieben kommt, so kann diese Abhängigkeit automatisch durch weitere Modifikationen erfüllt werden oder alternativ der Anwender darauf hingewiesen werden. Dieser Hinweis kann verschiedene Lösungsmöglichkeiten für die Erfüllung der Abhängigkeiten enthalten.

Nach jedem der Schritte 2400 und 2600 kann die gesamte Konfiguration auf Kompatibilität mit vorgegebenen Parametern in einem Schritt 2450, 2650, usw. geprüft werden. Diese Prüfung kann auch nach jeder Modifikation einer Rekonfiguration erfolgen. Mindestens nach Schritt 2600 sollte jedoch diese Prüfung stattfinden. Die Prüfung kann umfassen, die Prüfung des Gesamtgewichts sowie der Gewichte der einzelnen Siebe, Siebkörbe und Einhänger, die Prüfung ob alle notwendigen Instrumente enthalten sind, bzw. ob von jeder notwendigen Gruppe mindestens die notwendige Anzahl der Elemente enthalten sind, und die Überprüfung ob es für alle in einem Siebkorb angeordneten Elemente mindestens ein gemeinsames Reinigungs-, Desinfektions-, und Sterilisationsverfahren (RDS-Verfahren) gibt. Auch eine vollständige Überprüfung aller Abhängigkeiten ist vorstellbar, sowie Prüfungen auf weitere vorstehend diskutierte Anforderungen an ein Instrumentenset.

Im Anschluss an die Fertigstellung der Konfiguration können in Schritt 2700 anwenderspezifische Beschriftungen für die Siebe, Siebkörbe, Einhänger und auf den Sieben für die Instrumente sowie eine Farbkodierung der Siebe, Siebkörbe und Einhänger erfasst und integriert werden. Es können sowohl Standardinformationen sowie anwenderspezifische Informationen für die Beschriftung und Farbkodierung hinterlegt sein. Die Personalisierung der Instrumentensiebe, deren Inhalt und die Anordnung des Inhalts in den Sieben geschieht unter Berücksichtigung kundenspezifischer Vorgaben sowie unter Einhaltung der rechtlichen, regulatorischen und instrumentenspezifischen Vorgaben und Rahmenbedingungen.

In Schritt 2800 können Dokumentationsinformationen für die erstellte Konfiguration erstellt werden. Hierbei werden zur fertigen Konfiguration passend die notwendigen Informationen wie Anleitungen, Sieblisten, Packlisten, Packschablonen, Baupläne für zusammenzusetzende Instrumente, angepasste Operationsanleitungen, (angepasste) Gebrauchsanweisungen für Instrumente (engl. Instructions for use, IFU), ggf. Anzahl und Größen der notwendigen Sterilcontainer und weitere notwendige Informationen, wie z.B. vorgeschriebene Informationen zu Bestandteilen des Instrumentensets erstellt. Diese Informationen können angezeigt, zum Herunterladen angeboten und auch als Option der fertigen Konfiguration beigelegt werden. Die Operationsanleitungen werden ebenso, angepasst auf den gewählten Ablauf, die gewählte Patientenlagerung, den operativen Zugang und die benutzerdefinierten Instrumente, erstellt.

Abschließend kann die Konfiguration abgeschlossen werden und die Konfiguration im Sinne einer Bestellung weitergeleitet werden. Die Konfiguration kann dann im Schritt 2900 in eine Vorrichtung zur Zusammenstellung eingegeben werden, welche die in der Konfiguration enthaltenen Instrumente gemäß dieser Konfiguration in Siebe, Siebkörbe und Einhänger voll- oder teilautomatisiert zusammenstellt.

Im Folgenden werden mögliche Parameter, die für Instrumente und andere Elemente von Instrumentensets gespeichert werden können erläutert. Diese Parameter können bei der Berechnung von optimierten Konfigurationen für Instrumentensets berücksichtigt werden.

System: Dieser Parameter bestimmt das Implantatssystem, zu dem ein Instrument gehört.

Systemspezifische Informationen: Manche Instrumente, welche notwendig sind, um eine solche Implantat-OP durchzuführen, können für mehrere der verfügbaren Implantatssysteme verwendet werden, andere aber nicht.

Notwendige Anzahl: Dieser Parameter definiert, wie häufig ein Instrument in einem Siebkorb liegen muss.

Notwendigkeit: Dieser Parameter definiert in Abhängigkeit von einem Implantatssystem, ob ein Instrument in einer Konfiguration enthalten sein muss. In vielen Fällen muss mindestens ein Instrument dieses Typs im Set sein, jedoch kann eine Auswahl innerhalb einer Gruppe von Instrumenten erfolgen. Z.B. muss mindestens ein Handgriff aus der Gruppe der Handgriffe ausgewählt werden. Mit diesem Parameter kann sichergestellt werden, dass ein Set alle notwendigen Instrumente enthält, damit die Operation durchgeführt werden kann.

Gruppenzugehörigkeit: Mit diesem Parameter kann ein Instrument einer Gruppe zugewiesen werden, die bei einer Auswahl als Alternativen zur Verfügung stehen.

Gewicht: Die Gewichtsinformationen jedes Instruments sind wichtig, um daraus das Gesamtgewicht und die Siebgewichte des Instrumentensets zu ermitteln. Das Gesamtgewicht und die Siebgewichte unterliegen regulativen Vorgaben.

Bauraum und Spülraum: Der Parameter des Bauraumes kann verwendet werden, um sicherzustellen, dass die Instrumente in den Lagerungen auch gereinigt und sterilisiert werden können. Dabei ist es notwendig, bei der Anordnung der Instrumente im Siebkorb den notwendigen Bauraum zur Lagerung und Reinigung einzuhalten.

Navigiert: Dieser Parameter bestimmt, ob ein Instrument für eine navigierte Operation berücksichtigt werden muss oder nicht.

IFU: Dieser Parameter enthält eine Verknüpfung zu einer hinterlegten Gebrauchsanweisung, die verwendet werden kann um die Gesamtheit der Anleitungen zu erzeugen. Darüber kann auch sichergestellt werden, dass am Ende der Konfiguration eine Liste der Gebrauchsanweisungen aller im Set enthaltenen Instrumente vorliegt, die dem Set beigelegt werden müssen.

RDS: Über diesen Parameter wird, bevorzugt codiert, definiert, wie das jeweilige Instrument aufbereitet werden kann. Die codierte Form dieses Parameters kann z.B. ein 8-stelliger Code sein, aus dem ermittelt werden kann welche RDS-Verfahren auf dieses Instrument angewendet werden können. Für ein bestücktes Sieb muss am Ende eine Aufbereitungsmethode angegeben werden können, so dass es für alle in einem Siebkorb angeordneten Elemente mindestens ein gemeinsames Reinigungs-, Desinfektions-, und Sterilisationsverfahren (RDS-Verfahren) gibt.

Kombinationsgruppe: Dieser Parameter definiert, welche Instrumente zusammen kombiniert werden dürfen.

Zugang: Dieser Parameter definiert, für welche Art von operativem Zugang das jeweilige Instrument geeignet ist.

Patientenlagerung: Über diesen Parameter wird die Eignung von Instrumenten für die jeweilige Patientenlagerung definiert. Manche Instrumente sind speziell geeignet für den Einsatz bei Rückenlage, andere bei Seitenlage des Patienten. Entsprechend kann nach diesem Kriterium gefiltert werden.

Operationsphase: Für die Anordnung auf Basis der Reihenfolge der Instrumente kann über diesen Parameter bestimmt werden, in welcher Operationsphase ein Instrument zum Einsatz kommen kann. Dies ist besonders interessant für die Kombination der implantatsbezogenen und der standardchirurgischen Instrumente.

Produktkennzahl: Dieser Parameter definiert z.B. die Klasse der Medizinprodukte zu der das Instrument gehört. Je nach Klasse gelten andere regulatorischen Anforderungen, die bei der Zusammenstellung der Instrumente berücksichtig werden müssen.

Sieblayer: Über den Parameter Sieblayer wird hinterlegt, ob hinsichtlich der Anordnung im Siebkorb ein Instrument in einer zweiten Ebene liegen darf. Dies wäre z.B. nicht der Fall, wenn das Instrument die komplette Höhe eines Siebkorbs benötigt und deshalb unten liegen muss.

Layerraum: Dieser Parameter definiert, ähnlich zu Sieblayer, ob über oder unter dem jeweiligen Instrument ein anderes liegen darf.

Produktalter: Über diesen Parameter kann gespeichert werden, seit wann ein bestimmtes Instrument im System erfasst wird. Daraus ableitbar ist auch das Datum, zu dem ein Produkt sein Lebensende erreichen wird, so dass bei der Berechnung der Konfiguration berücksichtigt werden kann, ob das Produkt einzusetzen ist.

Geometrische Angaben: Bestimmte Instrumente unterscheiden sich durch geometrische Angaben. Bei einem Probenkopf oder Inlay kann z.B. der Kopf- oder Innendurchmesser eine Rolle spielen, so dass dieser hier hinterlegt werden kann. Gleichzeitig kann sichergestellt werden, dass die im Sieb enthaltenen Probeköpfe und Probeinlays auch kompatibel sind.

Kombinierbarkeit: Um sicherzustellen, dass nicht nur die Probeköpfe und Probeinlays kompatibel sind, sondern auch alle anderen enthaltenen Instrumente, vor allem auch über die verschiedenen Implantatssysteme hinweg, wird hier hinterlegt welche Instrumente untereinander kombiniert werden können.

Darüber hinaus können für die Instrumente weitere Informationen gespeichert werden, wie eine Beschreibung (auch in mehreren Sprachen), Artikelnummer, Produktbereich, Datum der letzten Änderung der Instrumenteninformationen etc. Diese Informationen können bei der Anzeige von Konfigurationen in grafischer Form oder in Textform wiedergegeben werden.

## Patentansprüche

1. Computer-implementiertes Verfahren (2000) zur Zusammenstellung von Instrumentensets für eine Operation, **dadurch gekennzeichnet, dass** das Verfahren (2000) die folgenden Schritte umfasst:
Eingeben von Konfigurationsparametern;
Berechnen (2400) von mindestens einer Konfiguration unter Berücksichtigung der Konfigurationsparameter;
Anzeigen (2500) der berechneten Konfigurationen;
Rekonfigurieren (2600) der berechneten Konfiguration im Ansprechen auf empfangene Eingaben durch Modifikationen die den Eingaben entsprechen; und
Prüfen (2650) der rekonfigurierten Konfiguration hinsichtlich vorab definierter Bedingungen.

2. Verfahren (2000) gemäß Anspruch 1, wobei das Eingeben von Konfigurationsparametern zumindest eines umfasst aus:
Anlegen einer neuen Konfiguration oder Laden einer gespeicherten Konfiguration (2100);
Eingeben (2200) von Konfigurationsparametern eine Patientenlagerung und oder eines medizinischen Zugangs betreffend; und
Auswählen (2300) mindestens eines Implantats für die Operation.

3. Verfahren (2000) gemäß Anspruch 1 oder 2, wobei das Verfahren (2000) weiterhin nach dem Berechnen (2400) und vor dem Anzeigen (2500) weiterhin den Schritt umfasst:
Prüfen (2450) der berechneten Konfiguration hinsichtlich vorab definierter Bedingungen.

4. Verfahren (2000) gemäß einem der Ansprüche 1 bis 3, wobei das Verfahren (2000) weiterhin nach dem Anzeigen (2500) und vor dem Rekonfigurieren (2600) weiterhin den Schritt umfasst:
Auswählen einer Konfiguration aus den berechneten Konfigurationen.

5. Verfahren (2000) gemäß einem der Ansprüche 1 bis 4, wobei das Verfahren (2000) weiterhin den Schritt umfasst:
Änderung (2700) von Beschriftung und Farbgebung des Instrumentensets oder Teilen davon.

6. Verfahren (2000) gemäß einem der Ansprüche 1 bis 5, wobei das Verfahren (2000) weiterhin den Schritt umfasst:
Erstellung (2800) von Dokumentationsinformationen für die Konfiguration.

7. Verfahren (2000) gemäß einem der Ansprüche 1 bis 6, wobei das Verfahren (2000) weiterhin die Schritte umfasst:
Weitergabe (2900) der Konfiguration zu einer Vorrichtung zur Zusammenstellung von Instrumentensets; und
Zusammenstellen des Instrumentensets.

8. System zur Datenverarbeitung, umfassend einen Prozessor, der so angepasst ist, dass er das Verfahren nach einem der Ansprüche 1 bis 7 ausführt.

9. Computerprogrammprodukt, umfassend Befehle, die bei der Ausführung des Programms durch einen Computer diesen veranlassen, das Verfahren nach einem der Ansprüche 1 bis 7 auszuführen.

10. Computerlesbares Speichermedium, umfassend Befehle, die bei der Ausführung durch einen Computer diesen veranlassen, das Verfahren nach einem der Ansprüche 1 bis 7 auszuführen.
